# EUROPEAN PATENT APPLICATION

(11) **EP 3 721 822 A1**
(43) Date of publication of application: **14.10.2020**
(21) Application number: 19168658.3
(22) Date of filing: 11.04.2019
(51) Int. Cl.: A61B 17/322

(54) **DEVICE FOR TAKING ESSENTIALLY TWO-DIMENSIONAL PLANE MATERIAL SECTIONS, TENSIONING DEVICE, SYSTEM FOR TENSIONING**

(71) Applicant: ETH Zurich, 8092 Zurich (CH)
(72) Inventor: Fox, Stephan, 8608 Bubikon (CH); GORT, Marcel, 4500 Solothurn (CH); FREY, Sonja, 8624 Grüt (Gossau ZH) (CH); HECKHORN GHILARDI, Manuel, 8055 Zürich (CH)

(57) **Abstract**

The invention concerns a device for taking essentially two-dimensional plane material sections, a tensioning device to tension an energy storage means as well as a system for tensioning an energy storage means. It also concerns a method of taking essentially two-dimensional plane material sections.

The device (10) for taking essentially two-dimensional plane material sections (11), in particular for taking skin tissue sections, comprises a fixation means (20) to be fixed to a surface of a material. The fixation means (20) comprises a fixation area (21) extending in a first plane (91). The device also comprises a support means (30) to be arranged adjacent to the fixation means (20) on the surface of the material and a movement means to realize a relative movement (200) of the fixation means (20) and the support means (30) in relation to each other essentially perpendicular to the first plane (91), so that a spacing (94) between a fixed material surface (12) fixed to the fixation means (20) and at least a section of its adjacent material surface (13) is achievable. The device further comprises a separation means (50) to achieve a separation operation (300) in a second plane (92) essentially parallel to the first plane (91), and an adjustment means adapted for adjusting the distance (95) between the first plane (91) and the second plane (92).

## Description

The invention concerns a device for taking essentially two-dimensional plane material sections, a tensioning device to tension an energy storage means as well as a system for tensioning an energy storage means. It also concerns a method of taking essentially two-dimensional plane material sections.

In multiple applications it is necessary to remove a section or piece of essentially two-dimensional plane material. The objective may be to remove defective sections of a material surface. Another objective may be to take samples of a material surface for further analysis or processing, for example in a laboratory. Such a requirement may exist for biologically active material as well biologically inactive material. Therefore, special equipment is necessary to take defined material samples with known size and thickness.

One special application in which it is necessary to take defined sections of a surface of a material is split-skin grafting, for example to serve skin wounds. An open wound is in many cases a risk for infection. A living being with an open wound also often suffers from serious pain and restrictions in movement. Therefore, quality of life is significantly reduced by open wounds. The objective is to cover and close those wounds quickly and effectively and thus promote the healing process. One known possibility is to use the skin of the living being itself to cover a wound. One area in which such a treatment is typically practiced is the care of burn wounds, in particular large burn wounds. For taking healthy skin to cover such wounds special equipments and devices that allow to take defined skin sections in an easy and fast way are necessary. However, the appropriate equipment is not available everywhere and is often associated with high costs in acquisition and/or operation.

Another important application in which it is necessary to take defined sections of a skin surface is the need of cells, in particular cells of the dermis or epidermis, to cure skin injuries or defects like chronical wounds or vitiligo. Also in this case, the use of split-skin samples is advantageous, in particular because wounds from a defined split-skin sampling in most cases recover to the previous state in aesthetic and physical structure.

Devices for taking skin sections are also known as dermatomes. The currently available dermatomes produce skin sections or skin grafts that are highly depending on the operator regarding the section's quality and in particular quality variation. In most cases surgeons adjust the final thickness of the material section by the angle at which they are holding the device and/or by the pressure they are applying. In the field of tissue engineering highly standardized samples are required in automated processes to achieve constant product quality.

In addition to skin grafting, there are a number of other fields of application in which defined samples of tissue and material surfaces are required.

Different devices were developed within the last years, in particular regarding skin grafting.

US 4 038 986 A shows a dermatome for cutting thin organic tissue sections for transplanting by using a thin resilient cutting band for cutting the surface. A T-shaped support member is adapted for positioning the cutting band and to determine the width of the section to cut. It also comprises a guide element to determine the thickness of the tissue section.

US 4 098 278 A also shows an apparatus and a method in which an adhesive strip with a given width and thickness is used in combination with a razor blade-type cutter. The adhesive strip is fixed to the skin. A sliding guide member which is carrying the blade is manually moved to remove a selected area of skin.

US 7 666 192 B2 shows a skin-harvesting device. The device shown there comprises a base plate with a window to allow the surgeon to see the portion of the skin to be harvested. The base plate has to be pressed against the skin. The thickness of the skin portion can be varied by a thickness plate coupled to a cutting frame comprising the blade. The blade is moved in an oscillating way while cutting and is powered by a motor.

CA 2943330 A1 shows a dermatome with a guiding element for an oscillating movement of a blade and a spacer for defining a cutting gap, wherein the guiding element and the spacer define a curved path. In particular, the said device is adapted to take skin grafts from curved surfaces like for example the head of a person.

The problem to be solved by the present invention is to provide a low-cost device and system for taking defined essentially two-dimensional plane material sections in an easy way, as well as a corresponding method.

The problem is solved by a device for taking essentially two-dimensional plane material sections according to claim 1, a tensioning device to tension an energy storage means according to claim 13, a system for tensioning an energy storage means according to claim 14 and a method of taking essentially two-dimensional plane material sections according to claim 15. Advantageous embodiments of the device are given in the subclaims 2 to 12.

A first aspect of the invention is a device for taking essentially two-dimensional plane material sections, in particular for taking skin tissue sections, comprising a fixation means to be fixed to a surface of a material, wherein the fixation means comprises a fixation area extending in a first plane. Also, the said device comprises a support means to be arranged adjacent to the fixation means on the surface of the material and a movement means to realize a relative movement of the fixation means and the support means in relation to each other essentially perpendicular to the first plane. Therefore, a spacing between a fixed material surface, that is fixed to the fixation means, and at least a section of its adjacent material surface is achievable. Furthermore, the device comprises a separation means to achieve a separation operation in a second plane that is essentially parallel to the first plane, and an adjustment means that is adapted for adjusting the distance between the first plane and the second plane.

In other words, the device is intended to separate sections of an essentially two-dimensional plane material, but not necessarily absolutely flat material, from the material adjacent to the section or surrounding it. The device allows to take standardized material sections, which means in particular standardized in thickness and planarity with low flatness deviation in the plane of its surface, with results essentially independent of the operator's experience.

The material may be skin tissue, in particular tissue of human skin or animal skin, but it is generally not excluded to take material sections of plants or inanimate objects.

In other words, one intended use of the device is to take standardized split-skin grafts. This special intended use of the device is also known as split-skin grafting or skin tissue biopsy. The device can, therefore, also be called a split-skin grafting device or a dermatome.

The fixation means is adapted to be fixed to a material surface like for example a skin tissue. The fixation means, therefore, comprises a fixation area.
In the intended use of the device the fixation area, which is located on the lower side of the fixation means that is facing the material surface, is arranged on the surface of the material and fixed there by forces of a physical or chemical nature like electrostatics, capillarity, magnetism, gravity, molecular attractions, van der Waals forces, adhesion, exertion of minor pressure, or others.

In other words, the fixation means can be a pad with an adhesive area on an essentially flat surface facing the material from which the section is to be taken.

The fixation area is extending in the first plane. The first plane is, according to the intended use of the device, defined by the lower surface of the fixation means, which is orientated to the material or tissue surface from which the section is to be taken.

The support means chiefly acts as a downholder, holding down the surface of the material adjacent to the surface of the material from which the section is to be taken. Its function is to hold down the surface of the material adjacent to the surface of the material that is fixed to the fixation area. Therefore, the support means comprises at least one support area, which is arranged parallel to the first plane and is defining a third plane. In other words, the support means, in particular its support area, is arranged in the vicinity of the fixation means. The support means may also serve several other functions of the device like for example being part of a covering element of the device.

In the intended use when the device is arranged on a material surface the third plane is lower than the first plane. Between the third plane and the first plane is a clearance. The clearance between the first plane and the third plane is defined by the arrangement of the fixation means and the support means at the end of the relative movement of both means perpendicular to the first plane. The fixation means and the support means are mechanically connected or connectable. The arrangement how these components are connected in the assembled state defines the clearance between the first plane and the third plane.

The movement means is a part of the device that enables the movement of the fixation means and the support means relative to each. The said movement is at least perpendicular to the first plane. It can also be adapted to realize a relative movement of both components in two directions in space at the same time or to realize a rotating movement of at least the support means as long as one component of the total movement results in a clearance between the third and the first plane. In other words, the relative movement lifts the surface fixed to fixation means in relation to its adjacent surface in a rising movement. Also, the movement stretches the material surface, in particular, the adjacent surface. Therefore, the support area is essentially surrounding the fixation area.

The separation means is a component of the device that is adapted to realize a separation operation. The separation operation requires a movement of the separation means or at least a part of the separation means.

The separation operation carries out the preferably total physical separation of at least the material that is fixed to the fixation area from its adjacent material

The separation operation can be realized by shearing as well as light cutting or other separating techniques.

The function of the adjustment means is to define the second plane. The second plane is the separation or cutting plane, which is essentially and preferably arranged parallel to the first plane and therefore parallel to the surface of the fixed material itself. The distance between the second plane and the first plane defines the thickness of the material to be taken. In other words, the adjustment means adjusts the graft thickness. The second plane is located between the first plane and the third plane. The adjustment means is working as a stopper means or abutment means regarding the separation means at least while the separation means is executing the separation operation. The adjustment means can be a part of the fixation means as well as of the support means and therefore build one physical unit with one of these means. It is also possible that the adjustment means is a separate and changeable component which includes the advantage that the sample thickness is adjustable.

The advantage of the device according to the invention is that it allows to take standardized material sections, in particular standardized in thickness and planarity with low flatness deviation in the plane of its surface, with results essentially independent of the operator's experience.
In one embodiment of the device according to the invention, the fixation area comprises at least partially an adhesive means adapted to adhesively fix the surface of the material to the fixation means.

The adhesive means may be an adhesive layer. The layer can be realized for example by a double-sided adhesive tape. It is also possible that the fixation area is at least partially coated with an adhesive substance. Adhesive coating can be realized for example by spraying an adhesive to the fixation area. It can be advantageous to first apply a second tape to the material surface from which the section is to be taken and then fix the fixation means onto this second tape.

In another embodiment of the device according to the invention, the device comprises a driving means to drive the separation means in order to execute the separation operation.

The driving means is preferably mechanically connected to the separation means. It drives the separation means by providing and/or transmitting a driving force to the separation means, so that the separation movement is achievable.

The separation movement is in particular a cutting movement of the separation means in one direction. The cutting should preferably be carried out in a steady uninterrupted movement. The fact that the driving means initiates and drives the movement and not the user himself, for example by guiding a knife or blade personally, creates a uniform and defined cutting movement.

The advantage of this embodiment is therefore that the driving force is controllable and not depending on the person using the device.

In another embodiment of the device according to the invention, the driving means comprises as an energy storage means at least one elastic element adapted to provide force to the separation means.

The force is provided by means of releasing mechanical energy which has been stored in the elastic element by elastic deformation. To transfer the mechanical energy the elastic element is preferably connected to the separation means.

In a further embodiment of the device according to the invention, the elastic element is at least one mechanical spring or a spring package.

In particular, the elastic element is a coil spring or helical spring. In general also a gas spring or a rubber spring are possibilities to realize the elastic element.

The elastic element can also be an elastic foam for example made of polyurethane.

The driving means, in particular when the device is comprising springs, can also comprise guidance elements like spring guides to determine position and direction of expansion and/or deformation of the elastic element. A guidance element has the advantage that the mechanical energy is released in the desired direction.

In addition a guidance element can guide the separation means while executing the separation operation as well and build the mechanical connection between the driving means and the separation means.

In another embodiment of the device according to the invention, the device comprises a release mechanism adapted for the release of the energy stored in the energy storage means in order to provoke the separation operation.

On one hand, the mechanism is adapted to hold the driving means, in particular the tensioned spring and thus the separation means, in the tensioned state, so that the device is prepared by storing energy for executing the separation operation. On the other hand, the mechanism is adapted to release the stored energy at a given time.

In another embodiment of the device according to the invention, the release mechanism comprises an operating element arranged on a surface of a device, facing away from the first plane and mechanically connected or mechanically connectable to the driving means.

In particular, the release mechanism comprises a release button that is mechanically connected to the separation means and/or the driving means of the device. Therefore, the operating element is in particular adapted to release the mechanical energy stored in an elastic element like for example a spring, and therefore the force needed to drive the separation means. In other words, the operating element is adapted to initiate or cause the separation movement when triggered by a person.

Advantageously the release mechanism comprises a locking mechanism for blocking the separation means as long as there is no connection to the fixation means. Therefore, the locking mechanism protects the user from injuries by unintended use. Advantageously, the release mechanism is adapted to realize a single-hand-use.

The separation means may comprise a cutting means, in particular a blade, adapted to cut mechanically through the material and/or the surface of the material, in particular essentially in the second plane.

The cutting edge is arranged in the second plane between the first plane, which is given by the fixation area and the third plane which is given by the support means. In a top view the blade is preferably a triangle shape. Other shapes of the blade are not excluded.

Advantageously, the separation means is geometrically designed in such a way that a high stiffness of the blade is supported.

In another embodiment of the device according to the invention, the adjustment means is formed by at least one protuberance of the fixation means extending beyond the first plane in the direction to the surface of the material. The protuberance is adapted to form a bearing surface for the separation means, in particular while executing the separation operation.

The bearing surface restricts a deflection of the separation means in a direction perpendicular to the second plane and, therefore, adjusts the distance between the first plane and the second plane while the separation operation is executed. In particular, the bearing surface restricts the separation operation in a direction perpendicular to the first plane to ensure sample thickness. In other words, the protuberance works as upper stopper or abutment for the separation means while moving through the tissue. By that the distance between the blade and the adjustment means ensures a precise and repeatable thickness and planarity.

The protuberance extends essentially perpendicularly to the first plane in the direction to the second plane.

Preferably, the adjustment means also extends parallel to the first plane like rails in the direction of the movement so that the whole separation operation is guided by the adjustment means with respect to the tissue sample thickness. This may be realized by the protuberances extending parallel along the lower side of the fixation means. It is also possible that the protuberance is chamfered on its side facing the starting point of the separation movement, so that the cutting means is guided to the second plane in the beginning of the operation from a starting position above the material surface.

Adjustment means and fixation means may be one physical unit. It is also possible that the adjustment means is changeable, so that sample thickness is modifiable.

For reasons of cleaning and/or to change the blade if it is no longer sharp enough the separation means may comprise an attaching element as well as a connecting mechanism to detachably connect the blade to the attaching element, so that the blade is exchangeable.

This does not exclude that the device may be adapted for a single use.

It is also advantageous if the movement means comprises a guide member adapted to guide the relative movement of the fixation means and the support means in relation to each other in a direction essentially perpendicular to the first plane.

That means that the lifting of the fixed material surface and the stretching of the adjacent surface is guided and therefore controlled.

In another embodiment the fixation means is supportable against the support means, at least in a state in which the fixation means is positioned to realize a spacing between the surface of the material fixed to the fixation means and at least a section of its adjacent surface. In a supported state a supporting force acts on the fixation means by a mechanical connection between the fixation means and the support means. The mechanical connection comprises a ramp that is formed between the fixation means and the support means. The device comprises at least one supporting element for supporting the ramp, wherein the ramp and the supporting element are positioned on the fixation means and the support means. The plane of the movement is essentially parallel to the first plane. Therefore, a spacing between the material surface fixed to the fixation means and at least a section of its adjacent surface is variable.

In other words, the fixation area and therefore the fixed material surface is raised in relation to the adjacent surface during the relative movement of the fixation means and the support means in the direction of the extension of the separation operation, resulting in the said spacing.

The geometry of the fixation means and support means is such that by sliding the support means over the fixation means, the fixation means is lifted and along with it the material surface attached to it. This can for example be realized by a notch and a pin, wherein the pin is inserted into the notch to realize the said mechanical connection. To realize a raising of the fixed material surface the notch, in particular a pin guiding surface of the notch, is not totally parallel to the first plane but inclining and therefore forming a ramp.

The ramp is not necessarily one coherent inclining surface. It is also possible that the ramp is realized by several separate surfaces in different height positions regarding the first plane.

It is possible that the ramp and the supporting element are formed by the fixation means and the support means.

By the principle of the inclining plane an angular deviation of up to 20° is realizable, wherein the angle is defined according to the first plane.

In another embodiment the ramp is positioned on or formed by the fixation means and the supporting element is located at or formed by the support means or vice versa.

In other words, the ramp can be formed by the fixation means, so that a relative movement of the support means that comprises the supporting elements leads to a raising of the fixed material surface.

Furthermore, the movement means comprises a stop member for limiting the relative movement of the fixation means and the support means in the direction perpendicular to the first plane, so that the fixation means and the support means are arrangeable in a defined position, respectively.

In other words, in the end of the relative movement of the fixation means and the support means the fixation means and the support means are arranged in a defined position. Under conditions of intended use this means that there is a defined distance between the support area of the support means, that defines the third plane, and the fixation area of the fixation means.

Also, the movement means may comprise a fixation member to mechanically fix the fixation means and the support means in a defined position relative to each other, in particular in the end of the relative movement.

In one embodiment the distance between the first plane and the second plane is at least 0.2 mm.

In particular, the sample thickness which is the distance between the first plane, that defines one side of the essentially two-dimensional plane material section, and the second plane, that defines the second side of the essentially two-dimensional plane material section, may vary between 0.2 mm and 1 mm.

The distance may be adjusted, for example, by different fixation means with protuberances of different vertical extension heights.

The spacing between the fixed material surface and its adjacent material surface which is the spacing between the third plane and the first plane is at least 15 mm.

The fixation area extends essentially parallel to the second plane in the direction of the separation operation at least 1 mm, preferred 5 mm, most preferred 10 mm, and crosswise to the direction of the separation operation also at least 101 mm, preferred 5 mm, most preferred 10 mm. In other words, the minimum material sample that is possible to take with the device according to the invention in 1x1 mm, preferably between 1x1 mm and 40x40mm. The size and the shape of the material sample can be adjusted by the form and arrangement of the fixation area and/or the adjustment means.

It is also possible that the device comprises a writeable surface, for example located on its cover adapted to be inscribed and/or marked with for example a sample number.

In another embodiment the device comprises a lower cover adapted to cover at least the fixation area and/or the material fixed to the fixation area.

The advantage of the lower cover is that it protects the fixation area and/or the material fixed to the fixation area against contamination or damage.

In another embodiment the device comprises a main cover wherein the cover is ergonomically designed for a single-hand-use.

Preferably, the device is made of materials that are rustproof, biocompatible, heat-resistant up to 200 °C and/or autoclavable.

A second aspect of the invention is a tensioning device to tension an energy storage means designed as a spring or spring package of the device according to the invention.

The tensioning device works as an energy transferring station, wherein it is adapted to transfer energy, in particular mechanical energy, to the energy storage means of the device. In other words, the tensioning device is adapted to deform and therefore tension the spring or the springs of a spring package and therefore prepare the device for the separation operation.

A third aspect of the invention is a system for tensioning an energy storage means designed as a spring or spring package of the said device that comprises a said tensioning device and at least one said device for taking essentially two-dimensional plane material sections according to the invention.

Another aspect of the invention is a method of taking essentially two-dimensional plane material sections, in particular for taking skin tissue sections, by means of the said device for taking essentially two-dimensional plane material sections, wherein the fixation means of the device is fixed to a surface of material by means of its fixation area, and wherein the support means is arranged adjacent to the fixation means. A relative movement of the fixation means and the support means in relation to each other essentially perpendicular to the first plane of the fixation means is realized. Therefore, a spacing between a material surface fixed to the fixation means and at least a section of its adjacent material surface is achieved. A separation operation in a second plane that is essentially parallel to the first plane, is realized by the separation means, wherein the distance between the first plane and the second plane is adjusted by an adjustment means.

The invention is explained below using the embodiment examples shown in the enclosed drawings.

It is shown in
- Fig. 1:: the arrangement and use of the device for taking essentially two-dimensional plane material sections according to the invention in three steps in a front view;
- Fig. 2:: the separation operation from a cross-sectional side view;
- Fig. 3:: the lower side of the fixation means with a fixed, essentially two-dimensional plane material section; and
- Fig. 4:: an embodiment of the device for taking essentially two-dimensional plane material sections as an explosion drawing.

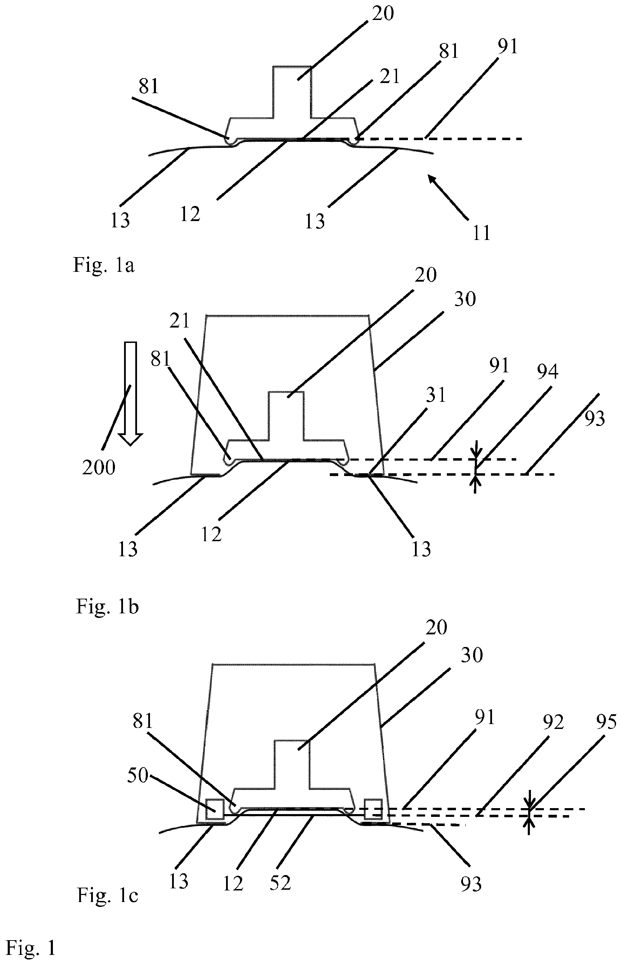

Figure 1 shows the arrangement of the device 10 for taking essentially two-dimensional plane material sections 11 according to the invention in three steps in a front view.

In the first step, shown in Figure 1a, the fixation means 20 is positioned on a material surface. On the lower side of the fixation means 20 in a first plane 91 a fixation area 21 is located. The fixation area 21 is in this embodiment adhesively fixed to the material surface. On its left side and its right side the fixation means 20 forms protuberances 81 on its lower side. The fixed material surface 12 between the protuberances 81 is the surface of the material section 11 to be taken by the method according to the invention.

The second step of the method of taking an essentially two-dimensional plane material section 11 is shown in Figure 1b. The support means 30 is arranged adjacent to the fixation means 20 on the surface of the material. By a relative movement 200 of the fixation means 20 and the support means 30 the fixed material surface 12 is lifted in relation to the adjacent material surface 13. In other words, the support means 30 holds down the adjacent material surface 13 by means of its support areas 31 and therefore a spacing 94 between the first plane 91 and the third plane 93 is created. At the same time the support means 30 stretches the adjacent material surface 13.

The third step is shown in Figure 1c. It is shown that the device comprises a separation means 50 to achieve a separation operation 300 in a second plane 92, that is essentially parallel to the first plane 91. After the spacing 94 is realized the separation operation 300 is executed by the separation means 50. Figure 1c shows the arrangement of fixation means 20, support means 30 and separation means 50 while taking the two-dimensional plane material section 11. It is shown that the blade 52 of the separation means 50 is located between the first plane 91 and the third plane 93 in a second plane 92. The distance 95 between the first plane 91 and the second plane 92 corresponds to the thickness of the material section 11 to be taken. It is shown that the thickness is given by the protuberances 81 the blade 52 abuts to. The extension of the protuberances 81 therefore adjust the sample thickness. The blade 52 moves in the direction of the plane of the Figure while cutting.

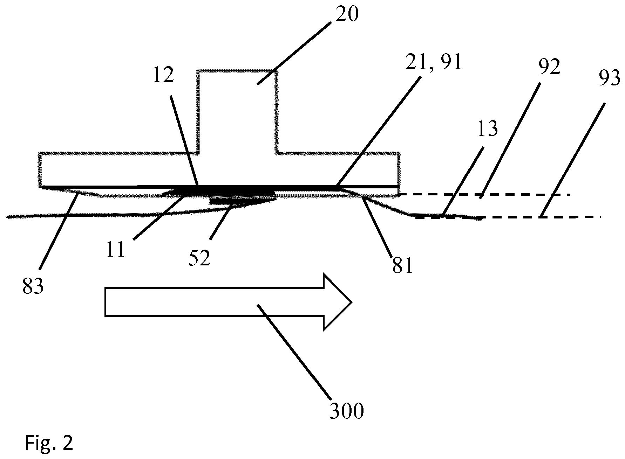

Figure 2 shows the separation operation 300. Compared to Figure 1 a cross-sectional righthand side view is shown. The arrow gives the direction of the separation movement 300 and therefore the direction of the movement of the blade 52 while cutting through the material parallel to the fixed material surface 12 fixed to the fixation area 21.The first plane 91 is shown in dotted lines. It can be seen that the separation operation 300 is half finished in Figure 2. The blade 52 is moving between first plane 91 and third plane 93, wherein the blade 52 is guided by the protuberances 81. It can also be seen that the protuberances 81 are chamfered on the left side where the movement of the blade 52 essentially starts. By the chamfers 83 the blade 52 is guided to the second plane 92 where the cutting through the material occurs. In the embodiment shown here the fixation means 20 combines the fixation function as well as the function of the adjustment means by comprising the protuberances 81.

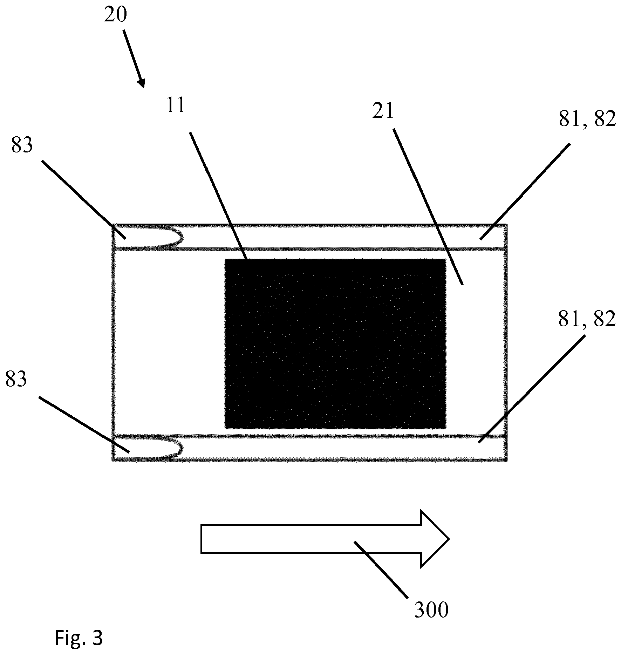

Figure 3 shows the lower side of the fixation means 20 with a fixed essentially two-dimensional plane material section 11 when the separation operation 300 is finished. In this Figure it can be seen that the protuberances 81 comprise bearing surfaces 82 to abut the blade 52. It also is shown that the protuberances 81 comprise chamfers 83. The chamfer guides the blade 52 into the second plane 92 in the beginning of the separation operation 300. The protuberances 81 are arranged on two sides of the fixation area 21.

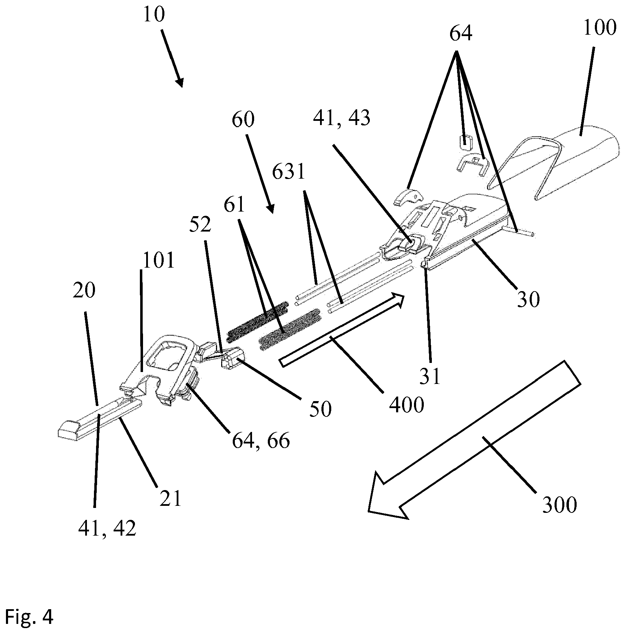

Figure 4 shows a special embodiment of the device 10 for taking essentially two-dimensional plane material sections 11 as an explosion drawing.

In this embodiment the support means 30 combines several functions of the device 10 according to the invention. Besides lifting the fixed material surface 12 and stretching the adjacent material surface 13 by the relative movement 200, it is a housing for the separation means 50 and the driving means 60 and is part of the release mechanism 64.

The driving means 60 that drives the separation operation 300 comprises in this embodiment four mechanical springs 63, arranged in two spring packages. Each spring 63 is assigned to a spring guide 631 which is arranged inside the spring 63 and is connected to the support means 30. The spring guides 631 are also assigned to the separation means 50 that comprises the blade 52. When the device 10 is prepared to execute the separation operation 300 the separation means 50 is moved along the spring guides 631 in the tensioning direction 400, wherein the mechanical springs 61 are tensioned and therefore mechanical energy is transferred to them. The preparation of the device 10 can be assured by a tensioning device.

The separation means 50 is held in this position with the tensioned mechanical springs 61 inside the body of the support means 30 by parts of the release mechanism 64. The separation means 50 can be released by the release button 66, so that the separation means 50 can slide along the spring guides 631 driven by the mechanical energy of the tensioned mechanical springs 61 in the direction of the separation operation 300.

The device 10 comprises a main cover 100 and a front cover 101. The shape of the main cover 100 is ergonomically adapted to the hand of a user. The device 10 is adapted for a single-hand use. If all components of the device 10 are arranged to each other according to the invention, the release button 66 extends beyond the front cover 101, so that it can be operated with one finger of the hand.

In the shown embodiment the supporting means 30 is assembled with the main cover 100, the front cover 101, the release mechanism 64 and the driving means 60 with its tensioned springs 61, as well as the separation means 50 to which the driving means 60 is mechanically connected.

In a first step for taking essentially two-dimensional plane material sections 11 by the device 10 according to this embodiment of the invention, the fixation means 20 is fixed by its in this embodiment adhesive fixation area 21 like shown in Figure 1a to the material section 11 to be taken.

In the second step the assembled supporting means 30 is arranged on the fixation means 20. For this purpose, the supporting means 30 is mechanically connected to the fixation means 20 by a shifting movement of the supporting means 30 in relation to the fixation means 20 essentially in the plane of the direction of the separation operation 300.

This shifting movement is carried out and guided by a guide member 41, comprising in this special embodiment a pin 42 and a notch 43. The pin 42 is located on the opposite side of the fixation area 21 of the fixation means 20. The notch 43 is formed by the support means 30. While arranging the fixation means 20 and the support means 30 by shifting both means in relation to each other the pin 42 is inserted into the notch 43. Therefore, the relative movement of fixation means 20 and support means 30 is guided by the guide member 41.

The pin 42 and the notch 43 form a ramp, so that, when the fixation means 20 and the support means 30 are shifted in the direction of the separation operation 300, the fixed material surface 12 is lifted by the relative movement 200 at the same time according to the principle of the inclined plane. Also, at the same time the adjacent material surface 13 is held down and stretched by the support means 30.

It is also possible that the guide member 41 is designed in such a way that the fixation means 20 and the support means 30 are arrangeable only by means of a movement vertical to the direction of the separation operation 300 to lift the fixed material surface 12.

**List of reference signs**

| | |
|---|---|
| device | 10 |
| material section | 11 |
| fixed material surface | 12 |
| adjacent material surface | 13 |
| fixation means | 20 |
| fixation area | 21 |
| support means | 30 |
| support area | 31 |
| guide member | 41 |
| pin | 42 |
| notch | 43 |
| separation means | 50 |
| blade | 52 |
| driving means | 60 |
| mechanical spring | 61 |
| spring guides | 631 |
| release mechanism | 64 |
| release button | 66 |
| protuberance | 81 |
| bearing surface | 82 |
| chamfer | 83 |
| first plane | 91 |
| second plane | 92 |
| third plane | 93 |
| spacing | 94 |
| distance | 95 |
| main cover | 100 |
| cover front | 101 |
| relative movement | 200 |
| separation operation | 300 |
| tensioning operation | 400 |

## Claims

1. Device (10) for taking essentially two-dimensional plane material sections (11), in particular for taking skin tissue sections, comprising a fixation means (20) to be fixed to a surface of a material, wherein the fixation means (20) comprises a fixation area (21) extending in a first plane (91), and a support means (30) to be arranged adjacent to the fixation means (20) on the surface of the material and a movement means to realize a relative movement (200) of the fixation means (20) and the support means (30) in relation to each other essentially perpendicular to the first plane (91), so that a spacing (94) between a fixed material surface (12) fixed to the fixation means (20) and at least a section of its adjacent material surface (13) is achievable, further comprising a separation means (50) to achieve a separation operation (300) in a second plane (92) essentially parallel to the first plane (91), and an adjustment means adapted for adjusting the distance (95) between the first plane (91) and the second plane (92).

2. Device (10) according to claim 1, **characterized in that** the fixation area (21) comprises at least partially an adhesive means adapted to adhesively fix the surface of the material to the fixation means (20).

3. Device (10) according to at least one of the preceding claims, **characterized in that** the device (10) comprises a driving means (60) to drive the separation means (50) in order to execute the separation operation (300).

4. Device (10) according to claim 3, **characterized in that** the driving means (60) comprises as an energy storage means at least one elastic element that is adapted to provide force to the separation means (50).

5. Device (10) according to claim 4, **characterized in that** the elastic element is at least one mechanical spring (61) or a spring package.

6. Device (10) according to at least one of the claims 4 to 5, **characterized in that** the device (10) comprises a release mechanism (64) adapted for release of the energy stored in the energy storage means in order to provoke the separation operation (300).

7. Device (10) according to claim 6, **characterized in that** the release mechanism (64) comprises an operating element arranged on a surface of the device, facing away from the first plane (91) and mechanically connected or mechanically connectable to the driving means (60).

8. Device (10) according to at least one of the preceding claims, **characterized in that** the separation means (50) comprises a cutting means, in particular a blade (52), adapted to cut mechanically through the material and/or the surface of the material, in particular essentially in the second plane (92).

9. Device (10) according to at least one of the preceding claims, **characterized in that** the adjustment means is formed by at least one protuberance (82) of the fixation means (20) extending beyond the first plane (91) in the direction to the surface of the material and that is adapted to form a bearing surface (82) for the separation means (50), in particular while executing the separation operation (300).

10. Device (10) according to at least one of the preceding claims, **characterized in that** at least in a state in which the fixation means (20) is positioned to realize a spacing (94) between the surface of the material fixed (12) to the fixation means (20) and at least a section of its adjacent surface (13), the fixation means (20) is supportable against the support means (30), wherein in a supported state a supporting force acts on the fixation means (20) by a mechanical connection between the fixation means (20) and the support means (30), and the mechanical connection comprises a ramp formed between the fixation means (20) and the support means (30), and the device (10) comprises at least one supporting element for supporting on the ramp, wherein the ramp and the supporting element are positioned on the fixation means (20) and the support means (30), so that a shifting of the fixation means (20) and the support means (30) in relation to each other according to the principle of the inclined plane is realizable by a movement of the ramp and the supporting element relative to each other in a movement plane that is essentially parallel to the first plane (91), so that a spacing (94) between the material surface fixed (12) to the fixation means (20) and at least a section of its adjacent surface (13) is variable.

11. Device (10) according to claim 10, **characterized in that** the ramp is positioned on or formed by the fixation means (20) and the supporting element is located at or formed by the support means (30) or vice versa.

12. Device (10) according to at least one of the preceding claims, **characterized in that** the distance (95) between the first plane (91) and the second plane (92) is at least 0.2 mm.

13. Tensioning device to tension an energy storage means designed as a mechanical spring (61) or spring package of the device (10) according to at least one of the preceding claims.

14. System for tensioning an energy storage means designed as a mechanical spring (61) or spring package of the device (10) according to at least one of the claims 1-12, comprising a tensioning device according to claim 13 and at least one device (10) according to at least one of the claims 1-12.

15. Method of taking essentially two-dimensional plane material sections (11), in particular for taking skin tissue sections, by means of the device (10) for taking essentially two-dimensional plane material sections (11) according to at least one of the claims 1 to 12, wherein the fixation means (20) of the device (10) is fixed to a surface of material by means of its fixation area (21), and wherein the support means (30) is arranged adjacent to the fixation means (20), and wherein a relative movement (200) of the fixation means (20) and the support means (30) in relation to each other essentially perpendicular to the first plane (91) of the fixation means (20) is realized, so that a spacing (94) between a material surface fixed (12) to the fixation means (20) and at least a section of its adjacent material surface (13) is achieved, and wherein a separation operation (300) in a second plane (92) essentially parallel to the first plane (91) is realized by the separation means (50) wherein the distance (95) between the first plane (91) and the second plane (92) is adjusted by an adjustment means.
